# EUROPÄISCHE PATENTSCHRIFT

(11) **EP 1 411 869 B1**
(45) Veröffentlichungstag und Bekanntmachung des Hinweises auf die Patenterteilung: **18.05.2005**
(21) Anmeldenummer: 01951306.8
(22) Anmeldetag: 31.07.2001
(51) Int. Cl.: A61F 2/34

(54) **KÜNSTLICHE GELENKPFANNE**
ARTIFICIAL JOINT PROSTHESIS
PROTHESE D'ARTICULATION ARTIFICIEL

(43) Veröffentlichungstag der Anmeldung: 28.04.2004
(73) Patentinhaber: Kuoni, Xaver, CH-8952 Schlieren (CH)
(72) Erfinder: KUONI, Xaver, CH-8952 Schlieren (CH); HOFER, Hannes, A-5020 Salzburg (AT)
(74) Vertreter: Schneider Feldmann AG Patent- und Markenanwälte
(86) Internationale Anmeldenummer: PCT/CH2001/000466
(87) Internationale Veröffentlichungsnummer: WO 2003/011196

(56) Entgegenhaltungen:
- EP-A- 0 923 915
- EP-A- 1 068 843
- EP-A- 1 072 236
- EP-A- 1 099 426
- WO-A-99/03429
- FR-A- 2 716 106
- US-A- 3 840 904

## Beschreibung

Die vorliegende Erfindung betrifft eine künstliche Gelenkpfanne gemäss Oberbegriff des Patentanspruches 1.

Künstliche Hüftgelenke umfassen üblicherweise eine Gelenkpfanne mit einem Einsatz, in welchen der Kopf eines Schafts eingreift. In Analogie zum ersetzten natürlichen Gelenk muss die künstliche Gelenkpfanne im Beckenknochen des Patienten positionsgenau und lagestabil anbringbar sein. Künstliche Hüftpfannen sind in verschiedenen Ausführungsvarianten bekannt. In der Praxis sind zwei Typen von Pfannen sehr gebräuchlich: konisch geformte Pfannen und sphärische Pfannen. Die Entscheidung für den einen oder den anderen Pfannentyp erfolgt je nach Indikation und Vorliebe des behandelnden Arztes.

Beide Pfannentypen umfassen jeweils eine metallische Aussenschale und einen in der Schale angeordneten Einsatz oder Inlay, der üblicherweise aus Keramik, Kunststoffen wie zum Beispiel Polyäthylen (Chirulen) oder Metall hergestellt ist. Solche künstliche Gelenkpfannen haben sich aufgrund ihrer Konstruktion aus Schale plus Einsatz als sehr verlässlich erwiesen.

Im folgenden soll vor allem auf sphärische Pfannen eingegangen werden. Ein Vorteil bei der Verwendung der sphärischen Pfannen liegt darin, dass beim Vorfräsen des sphärischen Betts weniger Beckenknochen entfernt werden muss als bei einer konisch geformten Pfanne, da das Acetabulum auch bei stark deformierten Gelenken annähernd sphärisch ist.

Zum Einfräsen eines Konus muss also immer mehr Knochen abgetragen werden als für ein sphärisches Bett. Auch das Fräsen selbst ist bei den sphärischen Pfannen einfacher, da die Richtung nicht so genau eingehalten werden muss. Die Gefahr dass Illium, Ischium oder Pubis durch eine zu tiefe Fräsung verletzt werden, ist viel beim sphärischen Bett viel geringer als beim konischen.

Neben der Standardausführung der sphärischen Pfannen, welche einer Halbkugel entspricht sind auch Pressfitausführungen sehr erfolgreich im Einsatz. Die Pressfitpfannen sind am Pol leicht abgeflacht und im Durchmesser etwas grösser als das vorgefräste halbkugelförmige Bett im Knochen. Dadurch wird der Presssitz der Pfanne im vorgefrästen Knochen gewährleistet. Eine rauhe Oberflächenbeschichtung der gebräuchlichen Schalen aus Metallen wie zum Beispiel Reintitan erleichtert das Anwachsen der Knochenzellen an das Implantat. Dies soll mittel- und langfristig eine optimale Sekundärstabilisierung sicherstellen. Die Pfanne kann auch mit Spongiosa hinterfüllt werden, um einen besseren Halt am Knochen zu erreichen. Die Primärstabilität wird üblicherweise, auch bei den Pressfitpfannen, durch mehrere zusätzliche Spongiosaschrauben oder andere Fixierungsmittel erhöht. Bei Schraubpfannen, wie sie zum Beispiel aus der EP-B-0'601'224 und der EP-A-0'943'304 und EP-A-0 923 915 bekannt sind, wird durch selbstschneidende Gewinde, welche auf der Aussenseite des Pfannengrundkörpers umlaufen, die Primärstabilität stark erhöht.

Im Gegensatz zu den Schraubpfannen werden die sphärischen Pfannen ohne Gewinde und vor allem die Pressfitpfannen in einer linearen Bewegung in das vorgefräste Bett eingeschlagen oder eingepresst.

Angepasst auf verschiedene Patientengrössen sind standardisierte Pfannengrössen von 44 bis 66, das heisst Pfannen mit einem Durchmesser der Pfannengrundfläche von 44 bis 66 mm, gebräuchlich und auf dem Markt erhältlich.

Um bei diesen Pfannentypen eine verbesserte Primärstabilität zu erreichen sind verschiedene Vorrichtungen vorgeschlagen worden. Aus der US-A-4'173'797 aus dem Jahre 1979 ist eine künstliche Gelenkpfanne mit einem basalen Flansch bekannt, der im eingesetzten Zustand an der Knochenoberfläche anliegen und dadurch ein Verkippen der Aussenschale verhindern soll. In dieser Veröffentlichung sind zusätzliche Stacheln im Polbereich der konvexen Schalenaussenseite beschrieben, die als Verdrehsicherung beim noch nicht eingewachsenen Implantat wirken sollen. Ein weiteres sphärisches Implantat mit stachelförmigen Projektionen im Polbereich zeigt die US-A-5'972'032. Die hier dargestellte Pressfitpfanne wird im wesentlichen entlang der Längs- bzw. Rotationsachse des Schalenkörpers eingeschlagen und durch die Dornen, welche im Polbereich im wesentlichen parallel zur Längs- bzw.

Rotationsachse des Implantates angeordnet sind, im eingeschlagenen Zustand drehsicher fixiert. Zur Sicherung gegen Zugbelastungen tragen diese Dornen jedoch nur bedingt bei. Es ist bekannt, dass sich bei Pressfitpfannen Druckkräfte mit einer signifikanten axialen Komponente im Hüftbein aufbauen können. Auch gegen diese Kräfte können die oben beschriebenen Dornen das Implantat nicht primär sichern.

Zwei weitere Lösungen zum Problem der ungenügenden Primärsicherung sind in den Publikationen US-A-5'755'799 und US-A-6'231'612 vorgeschlagen. Um eine schrauben- und zementlose Fixierung im Knochenbett zu ermöglichen, weisen beide Implantate im äquatorialen Bereich der Schalenaussenseite eine Vielzahl von kleinen Erhebungen auf.

Die Spitzen dieser schuppen- oder dachziegelartigen Widerhaken weisen bezogen auf den Patientenkörper nach distal, also entgegen die Einschlagrichtung.

Herstellungstechnisch bedingt folgen die Spitzen genau der Kontur der Mantelfläche und die einzelnen Schuppenreihen verlaufen von der Schalenöffnung in Polrichtung jeweils auf einer Linie parallel zur Einschlagrichtung. Die Herstellung solcher Implantate ist sehr aufwendig und damit kostenintensiv. Die nur wenige Zehntelmillimeter hohen Widerhaken verleihen dem frisch eingesetzten Implantat zudem oft nicht das gewünschte Mass an Primärstabilität.

Der Erfindung liegt deshalb die Aufgabe zugrunde eine künstliche Gelenkpfanne zur Verfügung zu stellen, die die genannten Nachteile nicht oder nur in geringerem Masse aufweist, wobei sie kostengünstig hergestellt werden kann und eine besonders einfache und exakte Implantation und eine zuverlässige Primärfixierung sicherstellt. Die optimale Wiederherstellung der anatomischen Pfannenfunktion des Hüftgelenkes mit physiologischer Krafteinleitung muss dabei ebenfalls gewährleistet sein.

Dies Aufgabe wird ausgehend von einer künstlichen Gelenkpfanne gemäss des Oberbegriffs des Anspruches 1, durch die kennzeichnenden Merkmale des Anspruchs 1 gelöst.

Die Erfindung umfasst die technische Lehre, dass sich eine künstliche Gelenkpfanne zum Einschlagen in ein vorgefrästes Bett im Knochen mit hervorragender Primärstabilisierung herstellen lässt, indem mindestens zwei Verriegelungselemente im distalen Mantelbereich der Pfannenschale angeordnet werden. Die Verriegelungselemente verankern die Pfannenschale im Hüftknochen und sichern diese gegen Zugkräfte, gegen Torsionskräfte und gegen kombinierte Zug- und Torsionskräfte. Die Verriegelungselemente gemäss der vorliegenden Erfindung erinnern auf den ersten Blick an Stege eines Steilgewindes.

Der Name Verriegelungselemente bringt aber schon zum Ausdruck, dass die Geometrie der erfindungsgemässen Verriegelungselemente dahingehend von üblichen Gewindestegen abweicht, dass sie ein Herausdrehen entgegen der Einschlagrichtung wirkungsvoll verhindern. Beim Einschlagen des Implantats schneiden sich die Verriegelungselemente in den Knochen ein und verdrehen die Pfannenschale um wenige Grad um die Pfannenachse. Da in einer besonders bevorzugten Ausführungsform die Steigung der stegförmigen Verriegelungselemente, bezogen auf die Pfannengrundfläche vom distalen oder äquatorialen zum proximalen oder polseitigen Ende hin zunimmt, verklemmen und verriegeln die Verriegelungselemente das Implantat gegen axiale Zugkräfte, radiale Torsionskräfte sowie Kombinationen von Beiden.

Weitere vorteilhafte Ausführungsvarianten ergeben sich aus der Beschreibung und den abhängigen Ansprüchen.

In den Zeichnungen sind Ausführungsbeispiele des Erfindungsgegenstandes dargestellt und in der nachfolgenden Beschreibung erläutert. Es zeigt:
- Figur 1a: eine Pressfitpfanne gemäss des Standes der Technik im Querschnitt, wobei nur die Schale gezeigt und das Inlay weggelassen ist;
- Figur 1b: die Pfanne gemäss Figur 1a in der Sicht von proximal in das Schaleninnere;
- Figur 2: eine Teilansicht einer Pressfitpfanne gemäss der vorliegenden Erfindung im Querschnitt im Bereich eines Verriegelungselements, wobei nur die Schale gezeigt und das Inlay weggelassen ist;
- Figur 3: eine Teilansicht einer Gelenkpfanne gemäss einer weiteren Ausführungsform der vorliegenden Erfindung von proximal, als vom Pol her gesehen;
- Figur 4: eine schematische Seitenansicht auf ein lineares Verriegelungselements;
- Figur 5a: eine Seitenansicht auf einen distalen Mantelbereich einer sphärische Pfanne mit einem Verriegelungselements gemäss Figur 3;
- Figur 5b: eine Seitenansicht auf einen distalen Mantelbereich einer sphärische Pfanne mit einer weiteren Ausführungsform eines Verriegelungselements;
- Figur 5c: eine Seitenansicht auf einen distalen Mantelbereich einer sphärische Pfanne mit einer weiteren Ausführungsform eines Verriegelungselements;

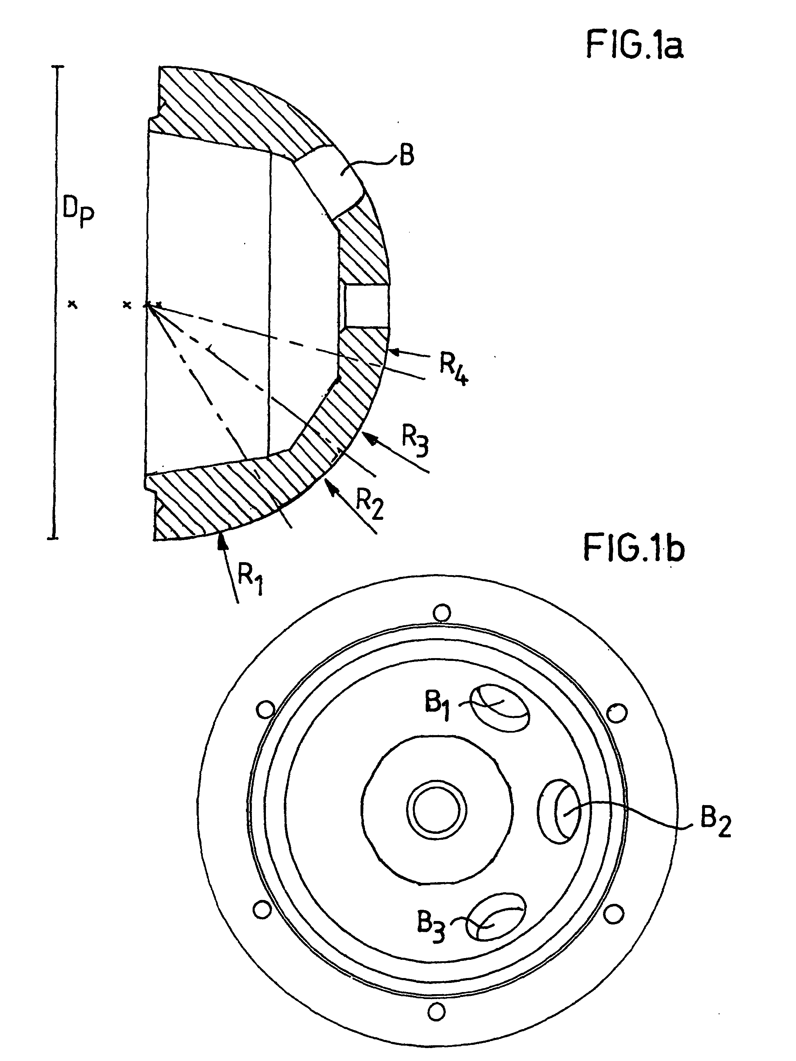

Figur 1a zeigt einen Halbschnitt durch einen Grundkörper oder eine Schale einer Pressfitpfanne gemäss des Standes der Technik, wie sie von der Anmelderin sehr erfolgreich produziert und vertrieben wird. Die Pressfitpfanne weicht durch ihre zunehmende Abflachung in Richtung Polbereich von der Halbkugelform ab. Der Grundkörper lässt sich, wie durch die strichlinierten Linien angedeutet ist, in vier Pfannenschichten einteilen. Die zugehörigen Mantelflächen weisen von distal, d.h. entfernt vom Pol, nach proximal, jeweils einen zunehmenden Radius auf. Die dargestellte Schnittebene enthält die Pfannenachse. Im Inneren weist die Schale eine Ausnehmung zur Aufnahme eines Keramik- oder Kunststoffinlays auf. Dieses, in der Figur nicht dargestellte Inlay dient als Widerlager für den Kugelkopf einer Femurprothese, eines sogenannten Schafts, zu liegen.

Anhand dieser bekannten Pressfitpfanne, soll dargestellt werden, dass bei den neuen Pfannen gemäss der vorliegenden Erfindung alle wesentlichen vorteilhaften und bewährten Konstruktionsmerkmale in Bezug auf Materialwahl, Oberflächenbeschichtungen, Pfannengeometrie beibehalten werden. Diese sind bekannt und sollen hier nicht nochmals erläutert werden. Ein Element der bekannten Pfannen, welches bei den neuen Pfannen weggelassen ist, sind die in der Figur 1b dargestellten Bohrungen B₁, B₂ und B₃ zur Aufnahme von Spongiosaschrauben, welche zur Zusätzlichen Primärstabilisierung des Implantates im Knochen dienen.

Die neu hinzugekommen Merkmale gemäss der vorliegenden Erfindung sollen nun im weiteren beschrieben werden.

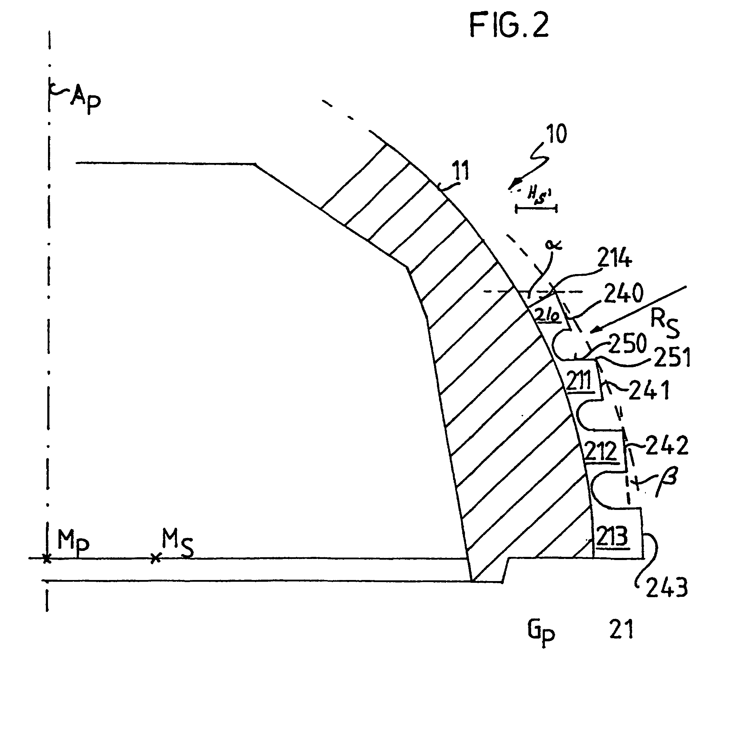

Die Figur 2 zeigt eine sphärische Pfanne 1, oder eine Pressfitpfanne 1 eines künstlichen Hüftgelenks im Halbschnitt. Die Pfannenachse A_{P} liegt in der Schnittebene. Ein Grundkörper oder eine Schale 10 weist eine zur Pfannenachse A_{P} im wesentlichen rotationssymmetrische, insbesondere sphärische oder ellipsoide Mantelfläche 11 auf. Das wesentliche neue Element ist das auf der Aussenseite des Grundkörpers 10 angeordnete Verriegelungselement 20. Im nur teilweise dargestellten Schnitt ist nur eines von mindestens zwei Verriegelungselementen 20 gezeigt. Das Verriegelungselement 20 umfasst Einschlagsteg 21, der im dargestellten Beispiel gezähnt ist.

Die zwei oder mehr Verriegelungselemente 20 sind symmetrisch über den Umfang der Pfanne (1) verteilt angeordnet, so dass sich die beim Einschlagen auftretenden Kräfte gleichmässig verteilen und die Pfanne nicht verkippt. In einer bevorzugten Ausführungsform sind die Verriegelungselemente 20 gleichmässig voneinander beabstandet angeordnet. Sind zum Beispiel drei Verriegelungselemente 20 vorhanden, heisst dies bezogen auf die kreisförmige Pfannengrundfläche G_{F}, dass je ein Element in Position 0°, 120° und 240° angeordnet ist. Bei vier Elementen wären es die Positionen 0°, 90°, 180°, 270° und 240°. Die Elemente 20 können auch ungleichmässig voneinander beabstandet angeordnet sein, solange die Symmetrie gewahrt ist. Das heisst, dass zum Beispiel vier Elemente in den folgenden Positionen angeordnet sein können: 0°, 60° und 180°, 240°.

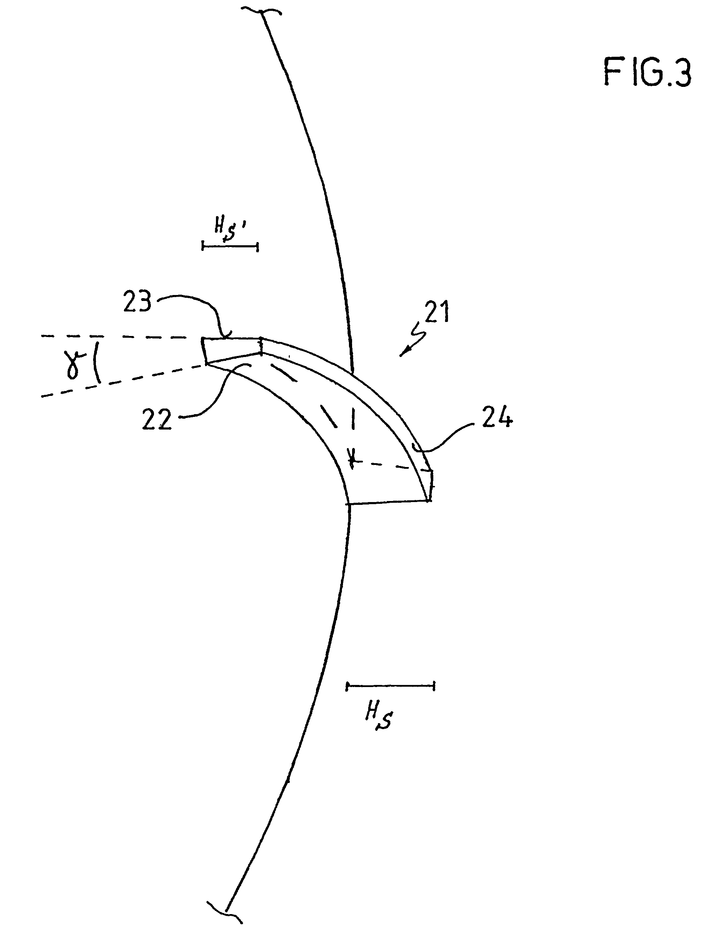

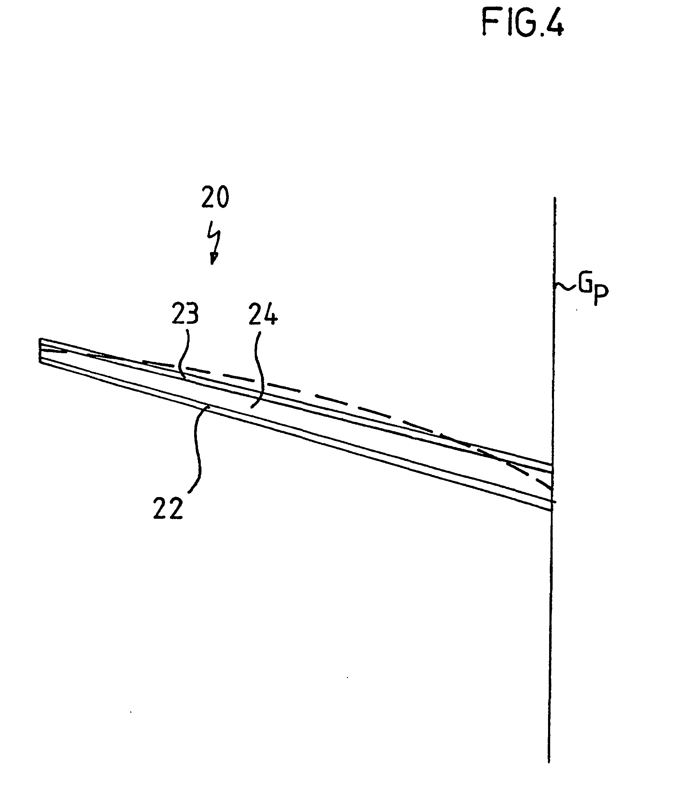

In der Figur 3 ist eine Teilansicht einer Gelenkpfanne gemäss einer weiteren Ausführungsform der Erfindung in einer Sicht vom Pol her dargestellt. Das gezeigte Verriegelungselement 20' umfasst einen Einschlagsteg 21, der eine besonders bevorzugte Geometrie aufweist. Die Steigung des Einschlagstegs 21 nimmt, bezogen auf die senkrecht zur Pfannenachse A_{P} liegende Pfannengrundfläche G_{P} vom distalen oder äquatorialen zum proximalen oder polseitigen Ende des Stegs 21 hin kontinuierlich zu. Die Figur 4 zeigt in einer Seitenansicht einer Gelenkpfanne einen einfacher gestalteten Steg 21, der mit gleichbleibender Steigung einen linearen verlauf nimmt. Strichliniert ist in Figur 4 die Kontur des Steges gemäss Figur 3 angedeutet, um die zunehmende Steigung des Einschlagstegs 21 aus der Figur 3 nochmals zu verdeutlichen. Vom Steganfang bis zum Ende weisen beide Stege eine Steigung von 75° bezogen auf die Grundfläche auf, was einem Drallwinkel von 15° entspricht. Die Steigung der Einschlagstege 21 kann vom distalen Steganfang bis zum proximalen Stegende zwischen 85° bis 60°, bevorzugt 80° bis 70°, und besonders bevorzugt 75°, bezogen auf die Grundfläche (G_{P}) betragen. Der Drallwinkel beträgt entsprechend 5° bis 30°, bevorzugt 10° bis 20°, und besonders bevorzugt 15°.

Die Krümmung, respektive die zunehmende Steigung des Steges gemäss der Figur 3, bezogen auf die führt dazu, dass die Pfannenschale nach dem Einschlagen im Hüftknochen verklemmt und verriegelt ist, so dass ein Herausdrehen entgegen der Einschlagrichtung wirkungsvoll verhindert wird. Die Verriegelungselemente 20 sichern das Implantat also äusserst wirkungsvoll gegen: i) axiale Zugkräfte, ii) radiale Torsionskräfte sowie iii) Kombinationen von Beiden.

Aus der Figur 3 ist ebenfalls ersichtlich, dass die Breite des Stegs 21 in bevorzugter Weise von der Stegbasis auf der Mantelfläche 11 über die gesamte Höhe des Steges hin abnimmt. Der dargestellte Steg ist gemäss einer bevorzugten Ausführungsform im Querschnitt trapezförmig und die Stegflanken 22, 23 konvergieren unter Einschluss eines Winkels γ von 15° bis 21°, vorzugsweise 18°, zueinander. Die Stegflanken können auch parallel zueinander ausgebildet sein, das Einschlagen hat sich jedoch mit den dargestellten trapezförmigen Stegen als einfacher erwiesen.

Unabhängig von der Grösse der Gelenkpfanne 1 beträgt die Steghöhe H_{S},H_{S'} zwischen 0.5 und 4 mm, vorzugsweise zwischen 1.8 und 2.6 mm. Diese Masse haben sich in Betracht auf die physiologischen Eigenschaften der Knochen, in die die Stege eingeschlagen werden als vorteilhaft erwiesen.

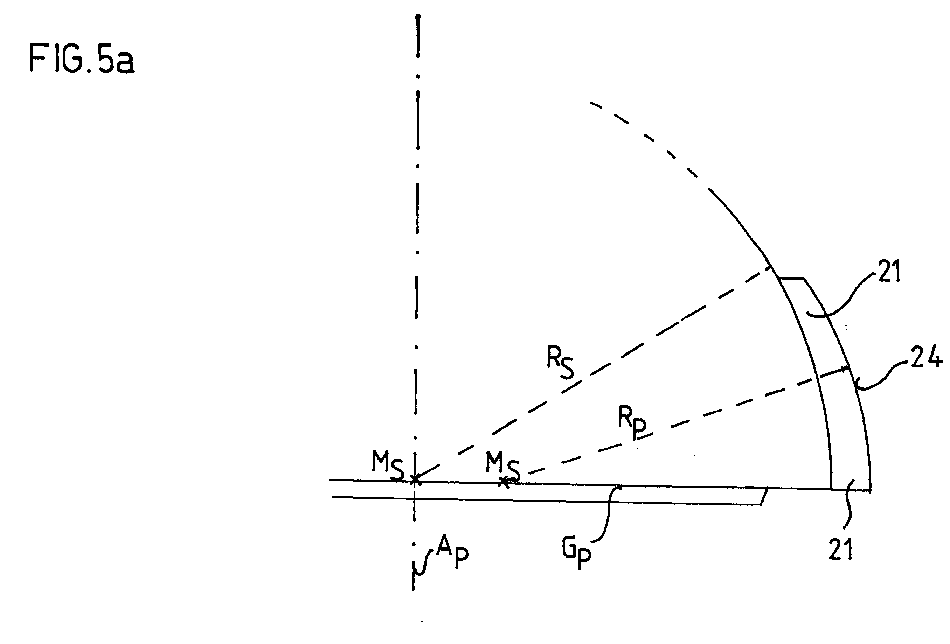

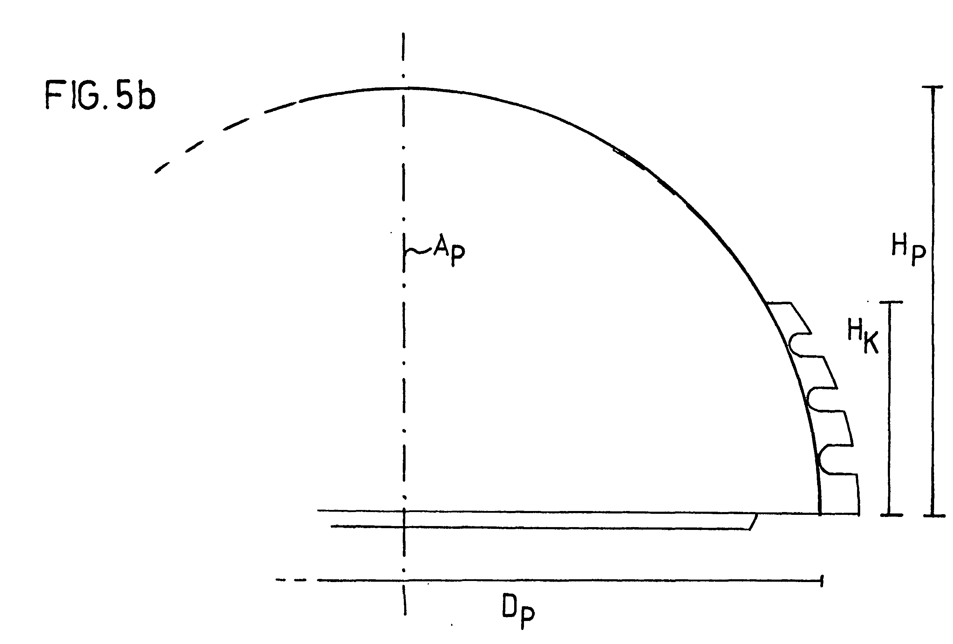

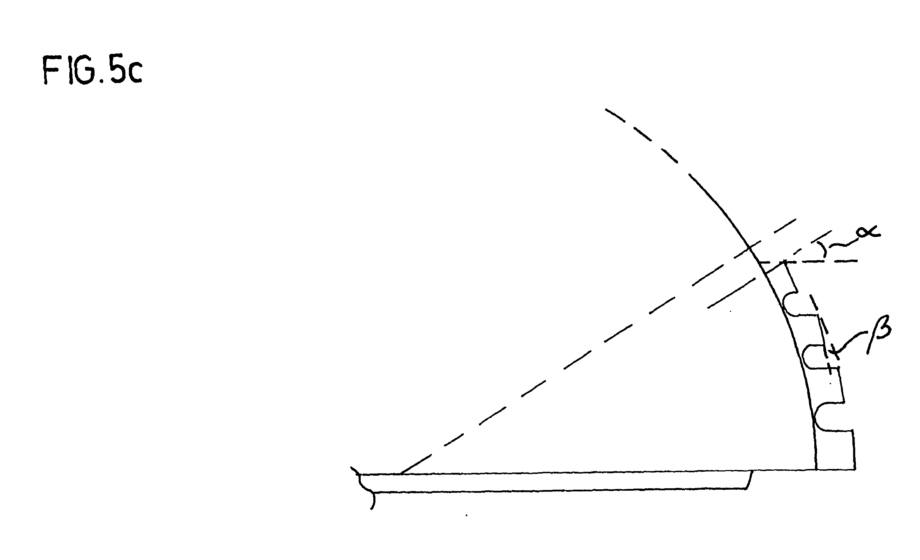

In der Figur 3 ist schon angedeutet, dass sowohl die Breite B_{S}, wie auch die Höhe H_{S} der Stege 21 vom distalen oder äquatorialen zum proximalen oder polseitigen Ende hin abnehmen. Ein Steg gemäss der Figur 3 ist in einer Seitenansicht in der Figur 5 dargestellt. Hier wird deutlich, dass ein Schneidenfläche 24 des Einschlagstegs 21 einem Kreisbogen mit einem Radius R_{S} folgt, welcher kleiner ist als der Mantelradius R₁ der korrespondierenden Kugelschicht, so dass die Verlängerung der Schneidfläche 24 mit der Mantelfläche 11 zusammenläuft. Die Steghöhe H_{S} am distalen oder äquatorialen Ende beträgt zwischen 1 und 4 mm, vorzugsweise 2.4 bis 2.8 mm. Am proximalen Ende beträgt die Steghöhe H_{S'} zwischen 0.5 und 3 mm, vorzugsweise 1.5 bis 1.8 mm. Die Höhe nimmt also vom distalen zum proximalen oder polseitigen Ende des Stegs hin kontinuierlich ab.

Der Steg 21 erstreckt sich ausgehend von der distalen Pfannengrundfläche G_{P} nur etwa bis zur halben Pfannenhöhe in Richtung Pol. Würde die Pfanne am proximalen Stegende gekappt, so bliebe eine distale Kugelschicht des Grundkörpers 10 übrig, deren Höhe H_{K} zwischen 20 bis 30%, vorzugsweise 24 bis 26%, des Pfannendurchmessers D_{P} entspricht. Da die Pfannen in ein vorgefrästes sphärisches Bett im Knochen eingeschlagen werden müssen, nimmt die Schneid- und

Klemmwirkung der Einschlagstege 21 mit zunehmender Entfernung von der Pfannengrundfläche G_{P} ab. Steganteile im Polbereich würden nicht mehr wesentlich zur erfindungsgemässen Sicherung des Implantates beitragen, sondern ähnlich den bekannten Dornen nur noch axial in den Knochen gequetscht werden.

Bei konischen Implantaten stellt ist die Situation anders gelagert. Hier können sich die Verriegelungselemente durchaus vorteilhafterweise über annähernd die gesamte Pfannenhöhe erstrecken.

In den Ausführungen der Figuren 2, 5b und 5c sind Gelenkpfannen 1 gezeigt, deren Verriegelungselemente keine durchgehenden, sondern mehrmals unterbrochene Stege 21 aufweisen, so dass Schneide- und Klemmzähne 210, 211, 212, 213 ausgeformt sind. Die Verzahnung erleichtert das Einschlagen der Implantate und stellt zusätzliche Fläche für ein späteres Anwachsen von Knochenzellen an den Stegen zur Verfügung. Ein weiterer Vorteil der Verzahnung besteht darin, dass sich die distalen Zahnrückseiten bei Zugbelastung das Implantat besonders effektiv im Knochen verriegeln.

Eine verbesserte Schneidwirkung beim Einschlagen wird dadurch erreicht, dass die oberen Schneidflächen 240, 241, 242 zumindest der proximalen Zähne 210, 211, 212 freigestellt sind. Die Schneidflächen 240, 241, 242 sind also um einen Freistellwinkel β in Bezug auf den Schneidenkreisbogen mit Radius R_{S} verkippt, so dass an der jeweiligen Stirnfläche 250 eines Zahnes 211 eine eigentliche Schneidkante 251 entsteht.

Zusätzlich zur Freistellung der proximalen Zähne ist in den Figuren 5c und 3 noch eine weitere Massnahme zum erleichterten Einschlagen verwirklicht. Der jeweils erste proximale oder polnächste Schneide- und Klemmzahn 213 eines Verriegelungselements 10 weist eine vordere Schneidfläche 214 auf, welche mit einem Spanwinkel α in Bezug auf die Pfannengrundfläche G_{P} angeordnet ist. Die vordere Schneidfläche 214 liegt also nicht parallel zur Pfannengrundfläche G_{P} sondern sie ist steiler angestellt und definiert vorzugsweise eine Ebene welche annähernd senkrecht auf der Mantelfläche 11 steht.

In den bisher beschriebenen Ausführungsformen der vorliegenden Erfindung waren die Verriegelungselemente 20 jeweils einstückig mit dem Grundkörper oder der Schale 10 geformt. In weiteren, in den Zeichnungen nicht dargestellten Ausführungsformen sind die Verriegelungselemente 20 an der Schale 10 lösbar befestigbar ausgebildet. Die bereits oben beschriebenen Einschlagstege 21 sind dabei auf einem Basis- oder Trägerelement angeordnet, das passgenau in eine entsprechende Nut in der Pfannenschale 10 einführbar ist. Werden die Verriegelungselemente 20 separat von dem Grundkörper 10 hergestellt, können bei Bedarf verschiedene Marterialkombinationen (Schale vs. Verriegelungselemente) zusammengestellt werden. Vorzugsweise werden die Verriegelungselemente vor dem Einschlagen in die entsprechenden Nuten im Grundkörper eingesetzt und gesichert. Es ist aber auch möglich, erst die Pfanne einzuschlagen und dann erst mit den separaten Verriegelungselementen 20 zu sichern.

## Patentansprüche

1. Gelenkprothese (1) mit einem Grundkörper (10) zum Einschlagen in einen Knochen **dadurch gekennzeichnet, dass** auf der Aussenseite des Grundkörpers (10) mindestens zwei Verriegelungselemente (20) angeordnet sind, welche jeweils mindestens einen Einschlagsteg (21) umfassen, welcher vom distalen Steganfang bis zum proximalen Stegende mindestens eine Steigung von 85° bis 60° bezogen auf die Grundfläche (G_{P}) definiert, was einem Drallwinkel von 5° bis 30° entspricht.

2. Pfanne (1) für ein künstliches Hüftgelenk mit einem Grundkörper oder einer Schale (10) der eine zur Pfannenachse (A_{P}) im wesentlichen rotationssymmetrische, insbesondere sphärische, ellipsoide oder konische Mantelfläche (11) aufweist, **dadurch gekennzeichnet, dass** auf der Aussenseite des Grundkörpers (10) mindestens zwei Verriegelungselemente (20) angeordnet sind, welche jeweils einen Einschlagsteg (21) umfassen, welcher vom distalen Steganfang bis zum proximalen Stegende mindestens eine Steigung von 85° bis 60° bezogen auf die Grundfläche (G_{P}) definiert, was einem Drallwinkel von 5° bis 30° entspricht..

3. Gelenkpfanne (1) nach Anspruch 2, **dadurch gekennzeichnet, dass** die Verriegelungselemente (20) symmetrisch über den Umfang der Pfanne (1) verteilt angeordnet sind.

4. Gelenkpfanne (1) nach Anspruch 3, **dadurch gekennzeichnet, dass** die Verriegelungselemente (20) gleichmässig voneinander beabstandet angeordnet sind.

5. Gelenkpfanne (1) nach Anspruch 2, **dadurch gekennzeichnet, dass** die Steigung der Einschlagstege (21) oder der stegförmigen Verriegelungselemente (21), bezogen auf eine senkrecht zur Pfannenachse (A_{P}) liegende Pfannengrundfläche (G_{P}) vom distalen oder äquatorialen zum proximalen oder polseitigen Ende der Stege (21) hin zunimmt.

6. Gelenkpfanne (1) nach Anspruch 2, **dadurch gekennzeichnet, dass** ein Schneidenradius (R_{S}) der Verriegelungselemente (21) kleiner ist als der Mantelradius (R₁) der korrespondierenden Kugelschicht, so dass die Verlängerung der Schneidfläche (24) mit der Mantelfläche (11) konvergiert.

7. Gelenkpfanne (1) nach Anspruch 2, **dadurch gekennzeichnet, dass** sich der Steg (20) über eine distale Kugelschicht des Grundkörpers (10) erstreckt, deren Höhe (H_{K}) zwischen 20 bis 30%, vorzugsweise 24 bis 26% des Pfannendurchmessers (D_{P}) entspricht.

8. Gelenkpfanne (1) nach Anspruch 2, **dadurch gekennzeichnet, dass** die Steghöhe (H_{S},H_{S'}) zwischen 0.5 und 4 mm, vorzugsweise zwischen 1.8 und 2.6 mm liegt.

9. Gelenkpfanne (1) nach Anspruch 8, **dadurch gekennzeichnet, dass** die Steghöhe (H_{S},H_{S'}) vom distalen oder äquatorialen zum proximalen oder polseitigen Ende der Stege (21) hin kontinuierlich abnimmt, wobei die Steghöhe (H_{S}) am distalen Ende zwischen 1 und 4 mm, vorzugsweise 2.4 bis 2.8 mm, und am proximalen Ende zwischen 0.5 und 3 mm, vorzugsweise 1.5 bis 1.8 mm beträgt.

10. Gelenkpfanne (1) nach Anspruch 2, **dadurch gekennzeichnet, dass** die Breite des Stegs (21) im Querschnitt von der Stegbasis auf der Mantelfläche (11) über die gesamte Höhe hin abnimmt

11. Gelenkpfanne (1) nach Anspruch 10, **dadurch gekennzeichnet, dass** der Steg im Querschnitt trapezförmig ist, wobei die Stegflanken (22, 23) zueinander konvergieren unter Einschluss eines Winkels γ von 15° bis 21°, vorzugsweise 18°.

12. Gelenkpfanne (1) nach einem der vorhergehenden Ansprüche, **dadurch gekennzeichnet, dass** Breite und Höhe der Stege (21) vom distalen oder äquatorialen zum proximalen oder polseitigen Ende hin abnehmen.

13. Gelenkpfanne (1) nach Anspruch 2, **dadurch gekennzeichnet, dass** der Steg (21) ein oder mehrmals unterbrochen ist, so dass er mindestens zwei Schneide- und Klemmzähne (210, 211, 212, 213) umfasst.

14. Gelenkpfanne (1) nach Anspruch 13, **dadurch gekennzeichnet, dass** die Zähne (210, 211, 212, 213) jeweils eine obere Schneidfläche (240, 241, 242, 243) umfassen, die zumindest teilweise mit einem Freistellwinkel β in Bezug auf den Schneidenkreisbogen mit dem Radius (R_{S}) angeordnet sind.

15. Gelenkpfanne (1) nach Anspruch 13, **dadurch gekennzeichnet, dass** der erste proximale oder polnächste Schneide- und Klemmzahn (213) eine vordere Schneidfläche (214) aufweist, welche mit einem Spanwinkel α in Bezug auf die Pfannengrundfläche (G_{P}) angeordnet ist.

16. Gelenkpfanne (1) nach Anspruch 2, **dadurch gekennzeichnet, dass** Verriegelungselemente (20) einstückig mit der Schale (10) geformt oder an der Schale (10) lösbar befestigbar ausgebildet sind.

17. Gelenkpfanne (1) nach Anspruch 2, **dadurch gekennzeichnet, dass** die Einschlagstege (21) vom distalen Steganfang bis zum proximalen Stegende eine Steigung von bevorzugt 80° bis 70°, und besonders bevorzugt 75°, bezogen auf die Grundfläche (G_{P}) definieren, was einem Drallwinkel von bevorzugt 10° bis 20°, und besonders bevorzugt 15° entspricht.

18. Pressfitpfanne nach einem der Ansprüche 2 bis 16.

## Claims

1. A joint prosthesis (1) with a base body (10) for knocking into a bone, **characterised in that** there are arranged at least two locking elements (20) on the outer side of the base body (10) which in each case comprise at least one knock-in web (21) which from the distal web beginning up to the proximal web end defines at least a gradient of 85° to 60° with respect to the base surface (G_{P}), which corresponds to an angle of twist of 5° to 30°.

2. A socket (1) for an artificial hip joint with a base body or a shell (10) which comprises an in particular spherical, ellipsoidal or conical superficies (11) essentially rotationally symmetrical to the socket axis (A_{P}), **characterised in that** on the outer side of the base body (10) there are arranged at least two locking elements (20) which in each case comprise a knock-in web (21) which from the distal web beginning up to the proximal web end defines at least a gradient of 85° to 60° with respect to the base surface (G_{P}), which corresponds to an angle of twist of 5° to 30°.

3. A joint socket (1) according to claim 2, **characterised in that** the locking elements (20) are arranged distributed symmetrically over the periphery of the socket (1).

4. A joint socket (1) according to claim 3, **characterised in that** the locking elements (20) are arranged uniformly distanced to one another.

5. A joint socket (1) according to claim 2, **characterised in that** the gradient of the knock-in webs (21) or of the web-like locking elements (21), with respect to a socket base surface (G_{P}) lying perpendicular to the socket axis (A_{P}), increases from the distal or equatorial end towards the proximal or pole-side end of the webs (21).

6. A joint socket (1) according to claim 2, **characterised in that** a cutting radius (R_{S}) of the locking elements (21) is smaller than the superficies radius (R₁) of the corresponding spherical layer so that the extension of the cutting surface (24) converges with the superficies (11).

7. A joint socket (1) according to claim 2, **characterised in that** the web (20) extends over a distal spherical layer of the base body (10) whose height (H_{K}) corresponds to between 20 to 30%, preferably 24 to 26% of the socket diameter (D_{P}).

8. A joint socket (1) according to claim 2, **characterised in that** the web height (H_{S}, H_{S'}) lies between 0.5 and 4 mm, preferably between 1.8 and 2.6 mm.

9. A joint socket (1) according to claim 8, **characterised in that** the web height (H_{S}, H_{S'}) reduces continuously from the distal or equatorial towards to proximal or pole-side end of the webs (21), wherein the web height (H_{S}) at the distal end is between 1 and 4 mm, preferably 2.4 to 2.8 mm, and at the proximal end between 0.5 and 3 mm. preferably 1.5 to 1.8 mm.

10. A joint socket (1) according to claim 2, **characterised in that** the width of the web (21) in cross section reduces from the base of the web on the superficies (11) over the whole height.

11. A joint socket (I) according to claim 10, **characterised in that** the web is trapezoidal in cross section, wherein the web flanks (22, 23) converge to one another enclosing an angle γ of 15° to 21 °, preferably 18°.

12. A joint socket (1) according to one of the preceding claims, **characterised in that** the width and height of the webs (21) reduce from the distal or equatorial towards the proximal or pole-side end.

13. A joint socket (1) according to claim 2, **characterised in that** the web (21) is interrupted once or several times so that it comprises at least two cutting and jamming teeth (210, 211, 212, 213).

14. A joint socket (1) according to claim 13, **characterised in that** the teeth (210, 211, 212, 213) in each case have an upper cutting surface (240, 241, 242, 243) which are arranged at least partly at a clearance angle β with regard to the cutting circular arc with the radius (R_{S}).

15. A joint socket (1) according to claim 13, **characterised in that** the first proximal or pole-closest cutting and jamming tooth (213) has a front cutting surface (214) which is arranged at an effective cutting angle α with regard to the socket base surface (G_{P}).

16. A joint socket (1) according to claim 2, **characterised in that** the locking elements (20) are formed as one piece with the shell (10) or are designed detachably fastenable to the shell (10).

17. A joint socket (1) according to claim 2, **characterised in that** the knock-in webs (21) from the distal web beginning up to the proximal web end define a gradient of preferably 80° to 70°, and particularly preferred 75° with respect to the base surface (G_{P}) which corresponds to a twist angle of preferably 10° to 20° and particularly preferred 15°.

18. A press fit socket according to one of claims 2 to 16.

## Revendications

1. Prothèse d'articulation (1) avec un corps de base (10) à implanter dans un os, **caractérisée par le fait qu'**au moins deux éléments de verrouillage (20) sont placés sur la face externe du corps de base (10), et ces éléments de verrouillage (20) comprennent respectivement au moins une âme d'implantation (21), laquelle définit, à partir du début distal de l'âme jusqu'à l'extrémité proximale de l'âme, une inclinaison de 85° jusqu'à 60° par rapport à la surface de base (Gp), ce qui correspond à un angle de torsion de 5° à 30°.

2. Prothèse (1) pour une articulation d'anche artificielle avec un corps de base ou une coque (10) qui présente une surface latérale (11) essentiellement à symétrie de révolution, en particulier sphérique, ellipsoïdal ou conique par rapport à l'axe de la prothèse (Ap), **caractérisée par le fait qu'**au moins deux éléments de verrouillage (20) sont placés sur la face externe du corps de base (10), et ces éléments de verrouillage (20) comprennent respectivement au moins une âme d'implantation (21), laquelle définit, à partir du début distal de l'âme jusqu'à l'extrémité proximale de l'âme, une inclinaison de 85° jusqu'à 60° par rapport à la surface de base (Gp), ce qui correspond à un angle de torsion de 5° à 30°.

3. Prothèse d'articulation selon la revendication 2, **caractérisée par le fait que** les éléments de verrouillage (20) sont placés de façon à être distribués symétriquement sur le périmètre de la prothèse (1).

4. Prothèse d'articulation selon la revendication 3, **caractérisée par le fait que** les éléments de verrouillage (20) sont placés de façon à être distanciés les uns des autres de manière uniforme.

5. Prothèse d'articulation selon la revendication 2, **caractérisée par le fait que** l'inclinaison des âmes d'implantation (21) ou des éléments de verrouillage (21) en forme d'âme, par rapport à une surface de base de la prothèse (G_{P}) étant située perpendiculairement par rapport à l'axe de la prothèse (A_{P}), augmente à partir de l'extrémité distale ou équatoriale de l'âme (21) vers l'extrémité proximale ou du côté polaire de l'âme (21).

6. Prothèse d'articulation selon la revendication 2, **caractérisée par le fait qu'**un rayon de coupe (R_{S}) des éléments de verrouillage (21) est plus petit que le rayon de l'enveloppe (R_{I}) de la zone d'une calotte sphérique correspondante, de sorte que le prolongement de la surface de coupe (24) converge avec la surface de l'enveloppe (11).

7. Prothèse d'articulation selon la revendication 2, **caractérisée par le fait que** l'âme (21) s'étend sur une zone d'une calotte sphérique distale du corps de base (10) dont la hauteur (H_{K}) correspond de 20 à 30%, de préférence de 24 à 26%, du diamètre de la prothèse (D_{P}).

8. Prothèse d'articulation selon la revendication 2, **caractérisée par le fait que** la hauteur de l'âme (H_{S}, H_{S'}) se situe entre 0.5 et 4 mm, de préférence entre 1.8 et 2.6 mm.

9. Prothèse d'articulation selon la revendication 8, **caractérisée par le fait que** la hauteur de l'âme (H_{S}, H_{S'}) diminue continuellement, à partir de l'extrémité distale ou équatoriale de l'âme (21) vers l'extrémité proximale ou du côté polaire de l'âme (21), auquel cas la hauteur de l'âme (H_{S}) s'élève à l'extrémité distale entre 1 et 4 mm, de préférence de 2.4 à 2.8 mm, et à l'extrémité proximale entre 0.5 et 3 mm, de préférence de 1.5 à 1.8 mm.

10. Prothèse d'articulation selon la revendication 2, **caractérisée par le fait que** la largeur de l'âme (21) diminue dans la section transversale, à partir de la base de l'âme sur la surface de l'enveloppe (11) sur l'ensemble de l'hauteur

11. Prothèse d'articulation selon la revendication 10, **caractérisée par le fait que** l'âme dans la section transversale est trapézoïdal, auquel cas les flancs de l'âme (22, 23) convergent l'un vers l'autre sous l'inclusion d'un angle γ de 15 jusqu'à 21°, de préférence 18°.

12. Prothèse d'articulation selon une des revendications précédentes, **caractérisée par le fait que** la largeur et la hauteur des âmes (21) diminuent à partir de l'extrémité distale ou équatoriale vers l'extrémité proximale ou du côté polaire.

13. Prothèse d'articulation selon la revendication 2, **caractérisée par le fait que** l'âme (21) est interrompue une ou plusieurs fois, de sorte qu'elle comprend au moins deux dents de serrage et coupantes (210, 211, 212, 213).

14. Prothèse d'articulation selon la revendication 13, **caractérisée par le fait que** les dents (210, 211, 212, 213) comprennent respectivement une surface de coupe supérieure (240, 241, 242, 243) qui est placée au moins en partie avec un angle de libération β par rapport à l'arc de cercle de coupe avec le rayon (R_{S}).

15. Prothèse d'articulation selon la revendication 13, **caractérisée par le fait que** la première dent de serrage et coupante (213) proximale ou prochainement polaire présente une surface de coupe (214) antérieure qui est placée avec un angle d'inclinaison α par rapport à la surface de base de la prothèse (G_{P}).

16. Prothèse d'articulation selon la revendication 2, **caractérisée par le fait que** des éléments de verrouillage (20) sont formés en une seule pièce avec la coque (10) ou alors sont développés de manière à être attachés de façon amovible à la coque (10).

17. Prothèse d'articulation selon la revendication 2, **caractérisée par le fait que** les âmes d'implantation (21) définissent, à partir du début distal de l'âme jusqu'à l'extrémité proximale de l'âme, une inclinaison de préférence de 80° à 70°, un montant de 75° étant particulièrement préféré, par rapport à la surface de base (G_{P}), ce qui correspond à un angle de torsion de 10° à 20°, un montant 15° étant particulièrement préféré.

18. Prothèse par technique d'ajustage serré selon une des revendications de 2 à 16.
